(19) Europäisches Patentamt
European Patent Office
Office européen des brevets

(11) **EP 3 750 866 B1**

(12) **EUROPÄISCHE PATENTSCHRIFT**

(45) Veröffentlichungstag und Bekanntmachung des Hinweises auf die Patenterteilung:
**09.02.2022  Patentblatt 2022/06**

(21) Anmeldenummer: **20178674.6**

(22) Anmeldetag: **08.06.2020**

(51) Internationale Patentklassifikation (IPC):
**C07C 67/03** (2006.01)    **C07C 29/149** (2006.01)
**C07C 67/38** (2006.01)    **C07C 69/24** (2006.01)

(52) Gemeinsame Patentklassifikation (CPC):
(C-Sets verfügbar)
**C07C 29/149; C07C 67/03; C07C 67/38**    (Forts.)

(54) **VERFAHREN ZUR HERSTELLUNG EINES ALKOHOLS AUS KOHLENWASSERSTOFFEN**

METHOD FOR MANUFACTURING ALCOHOL MADE OF HYDROCARBONS

PROCÉDÉ DE FABRICATION D'UN ALCOOL À PARTIR DES HYDROCARBURES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.06.2019  EP 19179572**

(43) Veröffentlichungstag der Anmeldung:
**16.12.2020  Patentblatt 2020/51**

(73) Patentinhaber: **Evonik Operations GmbH**
**45128 Essen (DE)**

(72) Erfinder:
• **KUCMIERCZYK, Peter**
**44652 Herne (DE)**
• **FRANKE, Robert**
**45772 Marl (DE)**
• **FRIDAG, Dirk**
**45721 Haltern am See (DE)**
• **KNOSSALLA, Johannes**
**46514 Schermbeck (DE)**
• **SCHÄPERTÖNS, Marc**
**45657 Recklinghausen (DE)**
• **GLUTH, Frederik**
**45472 Mülheim an der Ruhr (DE)**

(74) Vertreter: **Evonik Patent Association**
**c/o Evonik Industries AG**
**IP Management**
**Bau 1042A/PB 15**
**Paul-Baumann-Straße 1**
**45772 Marl (DE)**

(56) Entgegenhaltungen:
EP-A1- 0 937 698    EP-A1- 3 272 733
WO-A2-2012/074841    GB-A- 2 529 007
US-A1- 2009 012 323

(52) Gemeinsame Patentklassifikation (CPC): (Forts.)

C-Sets
**C07C 29/149, C07C 31/02;**
**C07C 67/03, C07C 69/24;**
**C07C 67/38, C07C 69/24**

**Beschreibung**

Die vorliegende Erfindung betrifft ein Verfahren zur Herstellung eines Alkohols durch Hydrierung eines Esters, der durch Alkoxycarbonylierung eines C2- bis C16-Kohlenwasserstoffs mit mindestens einer Mehrfachbindung, vorzugsweise mit mindestens einer olefinischen Doppelbindung, erhalten wird, bei dem das eingesetzte homogene Katalysatorsystem mittels Membrantrennung aus dem flüssigen Produktgemisch abgetrennt wird. In einer Weiterbildung der vorliegenden Erfindung wird der so gebildete Ester durch Umesterung zu einem anderen Ester umgesetzt und dann hydriert.

Die Herstellung von Alkoholen in der großindustriellen Chemie erfolgt zum größten Teil über die Hydroformylierung zur Herstellung eines Aldehyds mit nachfolgender Hydrierung des Aldehyds zum Alkohol. Wenngleich die Herstellung von Alkoholen im Wege der Hydroformylierung mit nachfolgender Hydrierung ein seit Jahrzehnten industriell etablierter und bewährter Prozess ist, weist er immer noch Verbesserungspotential auf. Ein Problem bei dieser Syntheseroute ist, dass bei der Hydroformylierung meistens hohe Drücke und hohe Temperaturen vorliegen und dadurch vergleichsweise hohe technische Anforderungen an die verwendeten Anlagen gestellt werden. Schließlich müssen die Anlagen die Drücke und Temperaturen aushalten können.

Ein weiteres grundsätzliches Problem der Herstellung von Alkoholen über Hydroformylierung und anschließender Hydrierung ist die hohe Reaktivität der als Zwischenprodukt anfallenden Aldehyde. Die Aldehyde sind sehr reaktiv und reagieren bereits vor der beabsichtigten Hydrierung zu unerwünschten Nebenprodukten weiter. Bei diesen unerwünschten Nebenprodukten handelt es sich meist um hoch siedende Substanzen, die aufwändig aus dem Reaktionsgemisch abgetrennt und entsorgt werden müssen. Zudem gehen durch die Hochsiederbildung ständig Aldehyde verloren, die nicht mehr zu den gewünschten Alkoholen umgesetzt werden können. Damit sinkt die Selektivität des Gesamtprozesses.

Der vorliegenden Erfindung lag daher die Aufgabe zu Grunde, einen alternativen Syntheseweg für die Herstellung von Alkoholen bereitzustellen, bei dem in der Carbonylierung ein geringerer apparativer Aufwand erforderlich ist. Eine weitere wichtige Zielsetzung ist, dass das Herstellungsverfahren im großindustriellen Maßstab durchführbar ist. Es gilt die klassische Synthese durch eine andere Synthesetechnologie zu ersetzen, welche die Produkte der klassischen Hydroformylierung in besserer Qualität liefert. Darüber hinaus soll der neu zu schaffende Syntheseweg weniger unerwünschte Nebenprodukte bilden.

In der US 2009/012323 A1 ist ein vergleichbares Syntheseverfahren beschrieben. Das Problem dabei ist aber, dass nach der Herstellung des Esters eine sich sofort anschließende Destillation durchgeführt wird, um den Ester aus der Produktmischung abzutrennen. Offensichtlich wird dabei die katalysatorhaltige Produktmischung der Destillation unterworfen. Eine solche thermische Behandlung kann jedoch dazu führen, dass der Katalysator desaktiviert wird oder sich sogar abscheidet und damit nicht mehr zu Verfügung steht. Gerade bei den typischerweise verwendeten Übergangsmetallen sind damit hohe Kosten verbunden, da die die aus dem Reaktor entfernt werden, ersetzt werden müssen. Die zugrundliegende Aufgabe war deshalb auch eine Verbesserung bekannter Verfahren durch Verringerung von Aufwand und Kosten.

Gelöst wird diese Aufgabe durch ein zweistufiges Verfahren in welchem in einem ersten Schritt in einer Alkoxycarbonylierung ein Kohlenwasserstoff mit mindestens einer Mehrfachbindung mit Kohlenmonoxid und einem Alkohol zu einem Ester umgesetzt wird und in welchem in einem zweiten Schritt in einer Hydrierung der Ester mit Wasserstoff zu dem gewünschten Zielalkohol umgesetzt wird. Dabei wird in dem zweiten Schritt der ursprünglich eingesetzte Alkohol teilweise wieder freigesetzt.

Erfindungsgemäß umfasst das Verfahren zur Herstellung eines Zielalkohols die folgenden Schritte:

a) Herstellung eines Esters durch Umsetzen (Carbonylieren) eines C2- bis C16-Kohlenwasserstoffs mit mindestens einer Mehrfachbindung mit Kohlenmonoxid und mit einem Alkohol A, welcher ein Mono- oder Polyalkohol (zwei oder mehr OH-Gruppen) mit 1 bis 50 Kohlenstoffatomen oder eine Mischung aus zwei oder mehr Mono- und/oder Polyalkoholen ist und welcher in einem Molverhältnis zum C2- bis C16-Kohlenwasserstoff (Alkohol: C2- bis C16-Kohlenwasserstoff) von 2 bis 20 eingesetzt wird, in Gegenwart eines homogenen Katalysatorsystems, welches zumindest ein Metall der Gruppe 8 bis 10 des Periodensystems der Elemente oder einer Verbindung davon, einen phosphorhaltigen Liganden und eine Säure umfasst, in einer Reaktionszone unter Erhalt eines flüssigen Produktgemisches;

b) Durchführen einer Membrantrennung zur Abtrennung des homogenen Katalysatorsystems aus dem flüssigen Produktgemisch, wodurch das homogene Katalysatorsystem und zusätzlich nicht umgesetzter Kohlenwasserstoff und/oder nicht umgesetzter Alkohol A im Retentat angereichert werden und der in Schritt a) gebildete Ester im Permeat angereichert wird, wobei als Membranmaterial ein säurestabiles, d. h. ein mindestens 300 h in Anwesenheit der Säure des Katalysatorsystems stabiles Membranmaterial ein OSN-fähiges (Organic Solvent Nanofiltration) Membranmaterial, welches zumindest eine trennaktive Schicht aufweist, eingesetzt wird und wobei das Retentat zur Reaktionszone in Schritt a) zurückgeführt und das Permeat zum nachfolgenden Schritt c) geführt wird;

c) Abtrennen des in Schritt a) gebildeten Esters aus dem Permeat in mindestens einem Trennverfahrensschritt, ausgewählt aus thermischer Trennung, beispielsweise Destillation, Extraktion, Kristallisation und Membrantrennung;

d) Hydrieren des in Schritt c) abgetrennten Esters mit Wasserstoff in Gegenwart des heterogenen Katalysatorsystems in einer Hydrierzone unter Erhalt eines Alkoholgemisches, welches zumindest den Zielalkohol, den abgespaltenen Alkohol A und nicht umgesetzte Ester umfasst;

e) Abtrennen des in Schritt d) gebildeten Zielalkohols in mindestens einem Trennverfahrensschritt, ausgewählt aus thermischer Trennung, beispielsweise Destillation, Extraktion, Kristallisation und Membrantrennung.

Die bei der Umsetzung in Schritt a) eingesetzten Kohlenwasserstoffe müssen mindestens eine Mehrfachbindung aufweisen. Bevorzugt sind Kohlenwasserstoffe mit mindestens einer olefinischen Doppelbindung, besonders bevorzugt sind Kohlenwasserstoffe mit einer olefinischen Doppelbindung. Grundsätzlich ist die Anzahl der Kohlenstoffatome von Verbindung, die mindestens eine Mehrfachbindung, vorzugsweise mindestens eine olefinische Doppelbindung aufweisen, nicht begrenzt. Technische relevant sind aber nur die Carbonylierung von C2- bis C20-Kohlenwasserstoffen mit mindestens einer Mehrfachbindung, vorzugsweise mindestens einer olefinischen Doppelbindung. In einer bevorzugten Ausführungsform der vorliegenden Erfindung können C3- bis C16-Kohlenwasserstoffe, besonders bevorzugt C4- bis C12-Kohlenwasserstoffe mit mindestens einer Mehrfachbindung, vorzugsweise mindestens einer olefinischen Doppelbindung eingesetzt werden. Darunter fallen insbesondere n-Alkene, iso-Alkene, Cycloalkene und aromatische Alkene mit 2 bis 20 Kohlenstoffatomen, vorzugsweise 3 bis 16 Kohlenstoffatomen, besonders bevorzugt 4 bis 12 Kohlenstoffatomen.

Die vorgenannt beschriebenen Kohlenwasserstoffe können zusätzlich zu der mindestens einen olefinischen Doppelbindungen eine oder mehrere weitere funktionelle Gruppen enthalten. Beispiele für geeignete funktionelle Gruppen sind Carboxyl-, Thiocarboxyl-, Sulfo-, Sulfinyl-, Carbonsäureanhydrid-, Imid-, Carbonsäureester-, Sulfonsäureester-, Carbamoyl-, Sulfamoyl-, Cyano-, Carbonyl-, Carbonothioyl-, Hydroxyl-, Sulfhydryl-, Amino-, Ether-, Thioether-, Aryl-, Heteroaryl- oder Silylgruppen und/oder Halogensubstituenten.

Besonders bevorzugte Kohlenwasserstoffe, die in Schritt a) des erfindungsgemäßen Verfahrens eingesetzt werden, weisen nur eine olefinische Doppelbindung auf, insbesondere n-Alkene und iso-Alkene mit 2 bis 20 Kohlenstoffatomen, vorzugsweise 3 bis 16 Kohlenstoffatomen, besonders bevorzugt 4 bis 12 Kohlenstoffatomen. Die eingesetzten Kohlenwasserstoffe sind vorzugsweise unsubstituiert.

Die eingesetzten und vorher beschriebenen Kohlenwasserstoffe mit olefinischer Doppelbindung werden erfindungsgemäß mit Kohlenmonoxid (CO) und einem Alkohol zum entsprechenden Ester in Schritt a) umgesetzt. Das Kohlenmonoxid kann dabei direkt als Einsatzgemisch oder durch die Zugabe eines Kohlenmonoxid-haltigen Gases, ausgewählt aus Synthesegas, Wassergas, Generatorgas und anderen Kohlenmonoxid-haltigen Gasen bereitgestellt werden. Möglich ist es auch, das Kohlenmonoxid dadurch bereitzustellen, dass das Kohlenmonoxid-haltige Gas vorher in einer für den Fachmann bekannten Weise in seine Komponenten aufgetrennt wird und das Kohlenmonoxid zur Reaktionszone geleitet wird. Das Kohlenmonoxid kann dabei weiterhin einen gewissen Anteil an Wasserstoff oder anderen Gasen enthalten, weil eine vollständige Auftrennung kaum zu realisieren ist.

Der bei der Umsetzung in Schritt a) eingesetzte Alkohol ist ein Mono- oder Polyalkohol (zwei oder mehr OH-Gruppen) mit 1 bis 50 Kohlenstoffatomen, vorzugsweise 1 bis 15 Kohlenstoffatomen, besonders bevorzugt 1 bis 10 Kohlenstoffatomen oder eine Mischung aus zwei oder mehr Monound/oder Polyalkoholen. In einer bevorzugten Ausführungsform ist der Polyalkohol ein Diol, Triol oder Tetrol, vorzugsweise ein Diol oder Triol mit der vorgenannten Anzahl an Kohlenstoffatomen. Geeignete Alkohole für die Umsetzung in Schritt a) sind Methanol, Ethanol, 1-Propanol, 1-Butanol, 1-Pentanol, 1-Hexanol, 2-Propanol, tert-Butanol, 3-Pentanol, 2-Propylheptanol, Cyclohexanol, Phenol oder Mischungen daraus.

Der in Schritt a) eingesetzte Alkohol wird, wenn es sich um einen Monoalkohol handelt, in einem Molverhältnis zum eingesetzten Kohlenwasserstoff (Monoalkohol : Kohlenwasserstoff) von 2 bis 20, vorzugsweise von 3 bis 10 und besonders bevorzugt von 4 bis 6 eingesetzt. Der Monoalkohol wird somit - bezogen auf den eingesetzten Kohlenwasserstoff - im molaren Überschuss hinzugefügt. Dadurch kann der Alkohol sowohl als Edukt für die Carbonylierung als auch als Lösemittel fungieren. Der in Schritt a) eingesetzte Alkohol wird, wenn es sich um einen Polyalkohol handelt, in einem Molverhältnis zum eingesetzten Kohlenwasserstoff (Kohlenwasserstoff : Polyalkohol) von 2 bis 20, vorzugsweise von 3 bis 10 und besonders bevorzugt von 4 bis 8 eingesetzt. Der Polyalkohol wird bezogen auf den eingesetzten Kohlenwasserstoff also im molaren Unterschuss hinzugefügt

Die erfindungsgemäße Umsetzung in Schritt a) wird in Gegenwart eines homogenen Katalysatorsystems durchgeführt. Bevorzugt umfasst das homogene Katalysatorsystem zumindest ein Metall aus der Gruppe 8 bis 10 des Periodensystems der Elemente (PSE) oder eine Verbindung davon, einen phosphorhaltigen Liganden und eine Säure als Co-Katalysator.

Das Metall aus der Gruppe 8 bis 10 des PSE ist vorzugsweise Palladium. Das Palladium wird bevorzugt in Form einer Vorläuferverbindung als Palladiumverbindung eingesetzt, die durch den phosphorhaltigen Liganden koordiniert wird. Beispiele für Palladiumverbindungen, die als Vorläuferverbindungen eingesetzt werden können, sind Palladiumchlorid [PdCl$_2$], Palladium(II)-Acetylacetonat [Pd(acac)$_2$], Palladium(II)-Acetat [Pd(OAc)$_2$], Dichloro-(1,5-cyclooctadien)palladium(II) [Pd(cod)$_2$Cl$_2$], Bis(dibenzylideneaceton)palladium(0) [Pd(dba)$_2$], Tris(dibenzylideneaceton)dipalladium(0) [Pd2(dba)$_3$] Bis(acetonitril)-dichloropalladium(II) [Pd(CH$_3$CN)$_2$Cl$_2$], Palladium(cinnamyl)-dichlorid [Pd(cinnamyl)Cl$_2$]. Vorzugsweise kommen die Verbindungen [Pd(acac)$_2$] oder [Pd(OAc)$_2$] zum Einsatz. Die Metallkonzentration von Palladium in Schritt a) beträgt vorzugsweise zwischen 0,01 und 0,6 Mol-%, bevorzugt zwischen 0,03 und 0,3 Mol-%, besonders bevorzugt zwischen 0,04 und 0,2 Mol-% bezogen auf die Stoffmenge des eingesetzten Kohlenwasserstoffs.

Geeignete phosphorhaltige Liganden des erfindungsgemäßen Katalysatorsystems weisen vorzugsweise eine Bidentat-Struktur auf. Bevorzugte phosphorhaltige Liganden für die das erfindungsgemäße Katalysatorsystem sind benzolbasierte Diphosphinverbindungen, wie sie beispielsweise in der EP 3 121 184 A2 offenbart worden sind. Die Liganden können in einer Vorreaktion mit dem Palladium kombiniert werden, sodass der Palladium-Ligand-Komplex zur Reaktionszone geführt wird, oder in situ zur Reaktion gegeben und dort mit dem Palladium kombiniert werden. Das Molverhältnis von Ligand : Metall kann für die beschriebene Umsetzung in Schritt a) 1 : 1 bis 10 : 1, vorzugsweise 2 : 1 bis 6 : 1, besonders bevorzugt 3 : 1 bis 5 : 1 betragen.

Das homogene Katalysatorsystem umfasst weiterhin eine Säure, wobei es sich insbesondere um eine Brönsted- oder eine Lewis-Säure handelt. Als Lewis-Säure können insbesondere Lewis Säuren mit einem LAU-Wert von mehr als 25, vorzugsweise mit einem LAU-Wert von 29. Der LAU-Wert ist eine neue Methode zur Bestimmung der Stärke von Lewis-Säuren (JR Gaffen et al., Chem, Vol. 5, Issue 6, S. 1567-1583). Als Lewis-Säure werden vorzugsweise Aluminiumtriflat, Aluminiumchlorid, Aluminiumhydrid, Trimethylaluminium, Tris(pentafluorophenyl)boran, Bortrifluorid, Bortrichlorid oder Mischungen daraus eingesetzt werden. Von den genannten Lewis-Säuren wird bevorzugt Aluminiumtriflat eingesetzt. Die Lewis-Säure wird vorzugsweise in einem Molverhältnis Lewis-Säure : Ligand von 1 : 1 bis 20 : 1, vorzugsweise 2 : 1 bis 15 : 1, besonders bevorzugt 5 : 1 bis 10 : 1 hinzugegeben.

Geeignete Brönsted-Säuren haben vorzugsweise eine Säurestärke von pKs ≤ 5, besonders bevorzugt eine Säurestärke von pKs ≤ 3. Die angegebene Säurestärke pKs bezieht sich auf den bei Normalbedingungen (25°C, 1,01325 bar) bestimmten pKs-Wert. Bei einer mehrprotonigen Säure bezieht sich die Säurestärke pKs im Rahmen dieser Erfindung auf den pKs-Wert des ersten Protolyseschrittes. Die Brönsted-Säure wird vorzugsweise in einem Molverhältnis Brönsted-Säure : Ligand von 1 : 1 bis 15 : 1, vorzugsweise 2 : 1 bis 10 : 1, besonders bevorzugt 3 : 1 bis 5 : 1 hinzugegeben.

Als Brönsted-Säuren können insbesondere Perchlorsäure, Schwefelsäure, Phosphorsäure, Methylphosphonsäure oder Sulfonsäuren eingesetzt werden. Geeignete Sulfonsäuren sind beispielsweise Methansulfonsäure, Trifluormethansulfonsäure, tert-Butansulfonsäure, p-Toluolsulfonsäure (PTSA), 2-Hydroxypropan-2-sulfonsäure, 2,4,6-Trimethylbenzolsulfonsäure und Dodecylsulfonsäure. Besonders bevorzugte Säuren sind Schwefelsäure, Methansulfonsäure, Trifluormethansulfonsäure und p-Toluolsulfonsäure. Vorzugsweise handelt es sich bei der Säure um Schwefelsäure.

Die Umsetzung bzw. Carbonylierung des eingesetzten Kohlenwasserstoffs mit olefinischer Doppelbindung in Schritt a) wird vorzugsweise bei einer Temperatur von 25 bis 140 °C, weiterhin bevorzugt bei einer Temperatur von 80 bis 130 °C und besonders bevorzugt bei einer Temperatur von 90 bis 120 °C durchgeführt. Der Druck in Schritt a) kann bei 5 bis 60 bar, vorzugsweise bei 10 bis 40 bar, besonders bevorzugt bei 15 bis 30 bar liegen.

Die beschriebene Umsetzung in Schritt a) findet in einer geeigneten Reaktionszone statt. Die Reaktionszone für die Umsetzung umfasst mindestens einen Reaktor, kann aber auch aus zwei oder mehr Reaktoren bestehen. Der mindestens eine Reaktor kann insbesondere aus der Gruppe, bestehend aus einem Rührkesselreaktor, einem Schlaufenreaktor, einem Jet-Loop-Reaktor, einem Blasensäulenreaktor oder Kombinationen daraus, ausgewählt sein. Sind mehrere Reaktoren vorhanden können die Reaktoren gleich oder unterschiedlich sein.

Durch die oben beschriebene Umsetzung in Schritt a) wird ein flüssiges Produktgemisch erhalten, die zumindest den durch die Umsetzung gebildeten Ester, das homogene Katalysatorsystem, nicht umgesetzte Alkohole A, und ggf. weitere Komponenten wie Leichtsieder, beispielsweise leichtsiedende Nebenprodukte wie Ether, und/oder Hochsieder und/oder nicht umgesetzte Kohlenwasserstoffe umfasst. Das flüssige Produktgemisch wird dann der nachfolgenden Membrantrennung in Schritt b) zugeführt. Bei der Umsetzung in Schritt a) kann zudem ein Abgas, welches zumindest aus unreaktiven Verunreinigungen wie Stickstoff, Wasserstoff und leichtsiedenden Nebenprodukten (beispielsweise die bereits genannten Ether) besteht, aus der Reaktionszone entnommen werden. Die Verunreinigungen und leichtsiedenden Nebenprodukte könnten sich akkumulieren und den Partialdruck des Reaktionsgases (CO) auf lange Sicht erniedrigen, wodurch die Reaktion verlangsamt werden könnte.

Im nachfolgenden Schritt b) wird das flüssige Produktgemisch einer Membrantrennung zugeführt, um das homogene Katalysatorsystem vom flüssigen Produktgemisch abzutrennen. Durch das erfindungsgemäße Membranmaterial werden das homogene Katalysatorsystem und nicht umgesetzter Kohlenwasserstoff und/oder nicht umgesetzter Alkohol im Retentat angereichert, während der in Schritt a) gebildete Ester im Permeat angereichert wird. Das Permeat, welches den gebildeten Ester enthält, wird dann zum nachfolgenden Schritt c) geführt. Das Retentat, welches das angereicherte homogene Katalysatorsystem umfasst, wird dann in die Reaktionszone in Schritt a) zurückgeführt. Bei der Rückführung des Retentats kann weiterhin ein Purgestrom, welcher inerte Alkane, leichtsiedende Nebenprodukte (bspw. Ether), mögliche Abbauprodukte des Katalysatorsystems oder andere Verunreinigungen enthalten kann, die durch die eingesetzten Kohlenwasserstoffströme eingetragen werden, wie beispielsweise Spuren von Wasser oder Stickstoff, entnommen werden, um eine Akkumulation in der oder den Reaktionszonen zu vermeiden. Die Rückführung des Retentats sorgt dafür, dass das bei der Membrantrennung im Retentat anfallende Katalysatorsystem zur Reaktion zurückgeführt wird. Dadurch werden Katalysatorverluste durch Abscheidung oder Desaktivierung (auftretend bspw. bei Destillationen) minimiert und das Verfahren kostengünstiger gestaltet. Katalysatorverluste sind meistens nicht ganz zu vermeiden, aber die erwähnte Verringerung der Verluste führt dazu, dass weniger Katalysator durch Zuführung von frischem Katalysator ersetzt werden muss.

Die Membrantrennung beruht auf der Semipermeabilität des Membranmaterials, welches für bestimmte Stoffe durchlässig und für andere Stoffe undurchlässig ist. Das in Schritt b) des erfindungsgemäßen Verfahrens eingesetzte Membranmaterial ist ein OSN-Membranmaterial (OSN = Organic Solvent Nanofiltration). Ein solches Membranmaterial besteht vorzugsweise zumindest aus einer eher dünnen trennaktiven Schicht (auch: aktiven Trennschicht) und optional einem dickeren Backing, worauf sich die trennaktive Schicht befindet. Bevorzugt besteht das erfindungsgemäße Membranmaterial zumindest aus einer trennaktiven Schicht und einem Backing. Zwischen trennaktiver Schicht und Backing können eine oder mehrere Zwischenschichten vorhanden sein. In einer bevorzugten Ausführungsform besteht das Membranmaterial nur aus der trennaktiven Schicht und dem Backing. Das Membranmaterial, aus zumindest trennaktiver Schicht und Backing, sollte säurestabil sein, damit das Membranmaterial nicht durch die im flüssigen Produktgemisch als Co-Katalysator befindliche Säure beschädigt wird. Der Begriff "säurestabil" meint im Rahmen der vorliegenden Erfindung, dass das Membranmaterial mindestens 300 h in Anwesenheit der Säure des Katalysatorsystems, insbesondere einer Brönsted-Säure mit einem $pKs \leq 5$, besonders bevorzugt mit einem $pKs \leq 3$ oder einer Lewis-Säure mit einem LAU-Wert von mehr als 25, vorzugsweise mit einem LAU-Wert von 29, stabil ist und nicht zerstört wird, wodurch die eigentliche Trennwirkung nicht mehr erzielt werden könnte.

Das Backing weist insbesondere eine poröse Struktur auf, die für das durch die trennaktive Schicht gelangte Permeat durchlässig ist. Das Backing hat eine stabilisierende Funktion und dient als Träger für die trennaktiven Schicht. Das Backing kann grundsätzlich aus jedem geeigneten porösen Material bestehen. Voraussetzung ist jedoch, dass das Material säure- und basenstabil ist. Das Backing kann auch aus dem gleichen Material bestehen wie die trennaktive Schicht.

Die erfindungsgemäße trennaktive Schicht besteht vorzugsweise aus einem PAEK-Polymer (Polyaryletherketon). PAEK zeichnet sich dadurch aus, dass innerhalb der Widerholungseinheit Arylgruppen abwechselnd über eine Etherfunktionalität und eine Ketonfunktionalität verknüpft sind. Eine erfindungsgemäß bevorzugte trennaktive Schicht besteht aus PEEK (Polyetheretherketon). Als trennaktive Schicht können insbesondere bevorzugt PEEK-Polymere mit einem Sulfonierungsgrad von weniger als 20%, besonders bevorzugt mit einem Sulfonierungsgrad von weniger als 10% eingesetzt werden. Die entsprechenden PEEK-Polymere und deren Herstellung sind in der WO 2015/110843 A1 oder in J. da Silva Burgal et al.; Journal of Membrane Science, Vol. 479 (2015), S. 105-116 beschrieben. Es hat sich überraschend gezeigt, dass dieses Material besonders stabil ist, insbesondere auch gegenüber der Säure als Co-Katalysator des homogenen Katalysatorsystems. Außerdem zeichnet sich das erfindungsgemäße PEEK-Material dadurch aus, dass es als trennaktive Schicht eingesetzt bevorzugt die gebildeten Ester durchlässt, während selbst die eingesetzten Eduktalkohole zumindest teilweise zurückgehalten werden und sich dadurch im Retentat anreichern. Dadurch kann die nachfolgende Aufarbeitung des restlichen flüssigen Produktgemisches wirtschaftlicher und nachhaltiger betrieben werden, weil verglichen mit bekannten Membranmaterialien weniger Alkohole abgetrennt werden müssen.

Die Membrantrennung in Schritt b) wird vorzugsweise Temperatur von 25 bis 100°C, weiterhin bevorzugt 30 bis 80°C und besonders bevorzugt 40 bis 70°C durchgeführt. Um das flüssige Produktgemisch auf die bei der Membrantrennung bevorzugte vorherrschende Temperatur zu bringen, kann das Produktgemisch gekühlt werden. Neben einer aktiven Kühlung unter Verwendung eines Kühlmediums, kann die Kühlung auch über einen Wärmetauscher erreicht werden, wodurch ein anderer Strom innerhalb des erfindungsgemäßen Verfahrens erhitzt wird. Optional ist auch ein Degasingschritt zwischen der Reaktionszone in Schritt a) und der Membrantrennung in Schritt b) vorhanden, um vorher leicht flüchtige Verbindungen wie Kohlenmonoxid und/oder restliche, über das Abgas nicht abgetrennte unreaktiven Verunreinigungen wie Stickstoff, Wasserstoff, Alkane und leichtsiedenden Nebenprodukten (beispielsweise die bereits genannten Ether) aus der flüssigen Produktmischung zu entfernen. Dabei wird das Produktgemisch erst unter den Partialdruck der gelösten Komponenten wie Kohlenmonoxid entspannt, so dass diese ausgasen, um dann wieder den Druck zu erhöhen, der in der Membrantrennung vorgesehen ist.

Der Transmembrandruck (TMP) kann in Schritt b) 10 bis 60 bar, vorzugsweise 15 bis 55 bar, besonders bevorzugt 20 bis 50 bar betragen (relativ). Der permeatseitige Druck kann dabei oberhalb des atmosphärischen Drucks bis 15 bar, vorzugsweise 3 bis 7 bar betragen, woraus sich anhand des TMP dann der retentaseitige Druck ergibt. In einer bevorzugten Ausführungsform sollte bei den Druckverhältnissen und insbesondere beim permeatseitigen Druck darauf geachtet werden, dass der Druck in Abhängigkeit von dem eingesetzten Kohlenwasserstoff, dem eingesetzten Alkohol und der vorhandenen Temperatur eingestellt wird, um eine Verdampfung nach dem Durchtritt durch die Membran zu vermeiden, da dadurch die gesamte Fahrweise instabil werden könnte. Gleiches gilt grundsätzlich auch für gelöste Komponenten wie Kohlenmonoxid, die optional durch den bereits genannten Degasingschritt entfernt werden können.

Die Ökonomie von Membrantrennverfahren kann sich stark nach der Lebensdauer der verwendeten Membranmaterialien bemessen, weshalb auch die Lebensdauer bzw. die Stabilität der Membran ein Kriterium für die Auswahl des geeigneten Membranmaterials sein kann. Dabei wird eine Mindestlebensdauer von etwa einem halben Jahr angenommen. Das kann insbesondere für Prozesse, wie dem vorliegenden Verfahren, bei denen das Produkt im Permeat angereichert wird, relevant werden, weil die erforderliche Membranfläche mit der Gesamtkapazität des Verfahrens steigt.

Das Permeat aus der Membrantrennung (Schritt b)) wird im nachfolgenden Schritt c) einem Trennverfahren ausgewählt aus der Gruppe, bestehend aus einer thermischen Trennung, beispielsweise Destillation, einer Extraktion, einer Kristallisation oder einer weiteren Membrantrennung, unterworfen, um den in Schritt a) gebildeten Ester vom restlichen Permeat abzutrennen. Das Trennverfahren ist vorzugsweise eine Destillation. Die entsprechenden Verfahrensbedingungen sind dem Fachmann bekannt.

Es kann vorkommen, dass bei dem in Schritt c) verwendeten Trennverfahren, insbesondere bei der Destillation, nicht nur der gebildete Ester aus dem Permeat abgetrennt wird, sondern auch die möglicherweise entstandenen Hochsieder, beispielsweise hochsiedende Nebenprodukte, die bei der Umsetzung in Schritt a) anfallen können. Um diese Hochsieder zu entfernen kann das erfindungsgemäße Verfahren einen Aufreinigungsschritt aufweisen, nämlich die Aufreinigung des gebildeten Esters durch Abtrennung des Esters von im Permeat enthaltenden Hochsiedern mittels thermischer Trennung, Extraktion, Kristallisation oder Membrantrennung. Bevorzugt wird ein thermisches Trennverfahren, besonders bevorzugt eine weitere Destillation eingesetzt, um die gebildeten Ester aufzureinigen. Die Verfahrensbedingungen sind dem Fachmann bekannt.

Das in Schritt c) erhaltene zu großen Teilen vom in Schritt a) gebildeten Ester befreite Permeat, welches mindestens nicht umgesetzte Alkohole und/oder nicht umgesetzte Kohlenwasserstoffe enthält, wird in einer bevorzugten Ausführungsform einer Recyclekomponentenabtrennung unterworfen. Dabei werden die nicht umgesetzten Alkohole und/oder nicht umgesetzten Kohlenwasserstoffe vom restlichen Permeat, insbesondere den dort enthaltenen Leichtsiedern, mittels thermischer Trennung, Extraktion, Kristallisation oder Membrantrennung abgetrennt. Bevorzugt wird ein thermisches Trennverfahren, besonders bevorzugt eine weitere Destillation eingesetzt, um die nicht umgesetzten Alkohole und/oder nicht umgesetzten Kohlenwasserstoffe vom restlichen Permeat abzutrennen. Die Verfahrensbedingungen sind dem Fachmann bekannt. Die dabei erhaltenen nicht umgesetzten Alkohole und/oder die nicht umgesetzten Kohlenwasserstoffe können dann in die Reaktionszone zurückgeführt werden.

Der nach dem erfindungsgemäßen Verfahren gebildete Ester kann in zwei weiteren Verfahrensschritten c1) und c2) umgeestert werden. Dabei wird der Teil des Esters, der dem in Schritt a) eingesetzten ersten Alkohol A entspricht, durch einen zweiten Alkohol B ausgetauscht. Diese Umesterung wird nach dem oben genannten Schritt c), optional nach dem möglichen Aufreinigungsschritt, durchgeführt und umfasst die folgenden Schritte:

c1) Umestern des in Schritt a) gebildeten Esters mit einem zweiten Alkohol B, wobei dieser zweite Alkohol B sich von dem im Schritt a) eingesetzten Alkohol A unterscheidet, in einer zweiten Reaktionszone unter Erhalt eines zweiten flüssigen Produktgemischs, welches zumindest den Ester mit dem zweiten Alkohol B, den abgespaltenen ersten Alkohol A und nicht umgesetzte zweite Alkohole B umfasst;

c2) Abtrennen des gebildeten Esters mit dem zweiten Alkohol B vom restlichen zweiten flüssigen Produktgemisch und insbesondere vom abgespaltenen ersten Alkohol A durch thermische Trennung und/oder mittels Membrantrennung und Rückführung des abgespaltenen ersten Alkohols A in die Reaktionszone aus Schritt a), sowie Rückführung des nicht umgesetzten Alkohols B in die zweite Reaktionszone.

In Schritt c1) erfolgt die eigentliche Umeesterung, also die Abspaltung des eigentlich in Schritt a) angebundenen ersten Alkohols A und die Anbindung des zweiten Alkohols B. Dazu wird der in Schritt a) gebildete Ester in einer Reaktionszone mit einem zweiten Alkohol B, der sich vom ersten Alkohol A unterscheidet, umgesetzt. In einer besonders bevorzugten Ausführungsform ist der bei der Umesterung eingesetzte zweite Alkohol B im Vergleich zu dem in Schritt a) eingesetzten ersten Alkohol A ein höhersiedender Alkohol. Der zweite Alkohol B wird bei der Umesterung vorzugsweise im Überschuss zugegeben, um die Umesterungsreaktion zu begünstigen.

Der bei der Umesterung in Schritt c1) eingesetzte zweite Alkohol ist vorzugsweise ein Mono- oder Polyalkohol (mehr als 2 OH-Gruppen) mit 1 bis 50 Kohlenstoffatomen, weiterhin bevorzugt mit 1 bis 15 Kohlenstoffatomen, besonders bevorzugt mit 1 bis 10 Kohlenstoffatomen oder eine Mischung aus zwei oder mehr Mono- und/oder Polyalkoholen, mit der Maßgabe, dass der in Schritt a) eingesetzte erste Alkohol A und der zweite Alkohol B nicht identisch sind. In einer bevorzugten Ausführungsform ist der Polyalkohol ein Diol, Triol oder Tetrol, vorzugsweise ein Diol oder Triol mit der vorgenannten Anzahl an Kohlenstoffatomen. Geeignete Alkohole für die Umsetzung in Schritt a) sind Methanol, Ethanol, 1-Propanol, 1-Butanol, 1-Pentanol, 1-Hexanol, 2-Propanol, tert-Butanol, 3-Pentanol, 2-Propylheptanol, Cyclohexanol, Phenol, Pentaerythrit, Neopentylglykol, Trimethylolpropan, Dipentaerytriol oder Mischungen daraus, vorzugsweise Ethanol, 1-Propanol, 1-Butanol, 1-Pentanol, 1-Hexanol, 2-Propanol, tert-Butanol, 3-Pentanol, 2-Propylheptanol.

Die Umesterung in Schritt c1) wird vorzugsweise säure- oder basenkatalysiert durchgeführt. Als Säuren können Brönsted- oder Lewis-Säuren eingesetzt werden.

Geeignete Brönsted-Säuren für die Umesterung in Schritt c1) sind Perchlorsäure, Schwefelsäure, Phosphorsäure, Methylphosphonsäure oder eine Sulfonsäure, beispielsweise Methansulfonsäure, Trifluormethansulfonsäure, tert-Butansulfonsäure, p-Toluolsulfonsäure(pTSA), 2-Hydroxypropan-2-sulfonsäure, 2,4,6-Trimethylbenzolsulfonsäure oder Dodecylsulfonsäure. Vorzugsweise werden Schwefelsäure oder eine Sulfonsäure als Brönsted-Säure eingesetzt, besonders bevorzugt Schwefelsäure. Es können auch Metall oder deren Verbindungen eingesetzt werden, beispielsweise Zinnpulver, Zinn(II)oxid, Zinn(II)oxalat, Titansäureester wie Tetraisopropylorthotitanat oder Tetrabutylorthotitanat sowie Zirkoniumester wie Tetrabutylzirkonat sowie Natriummethanolat und Kaliummethanolat.

Geeignete Lewis-Säuren für die Umesterung in Schritt c1) sind Titan(IV)-isopropoxid, $Bu_2SnO$, BuSn(O)OH oder Aluminiumtriflat. Bevorzugt eingesetzte Lewis-Säuren sind Titan(IV)-isopropoxid, und Aluminiumtriflat

Geeignete Basen für die Umesterung in Schritt c1) sind Alkalimetalle, Alkalialkoxide, Alkali- oder Erdalkaliacetate oder -oxide, Alkali- oder Erdalkalialkoholate, wie NaEtOH oder MgEtOH oder Alkalicarbonate, wie $K_2CO_3$ oder $CS_2CO_3$. Es können aber auch basische Ionenaustauscher oder NaOH eingesetzt werden. Bevorzugt werden Na- oder Mg-Alkoholate, wie NaEtOH oder MgEtOH, eingesetzt.

Die säurekatalysierten Umesterung wird vorzugsweise bei einer Temperatur von 60 bis 220 °C, weiterhin bevorzugt von 100 bis 210 °C und besonders bevorzugt bei 130 bis 200 °C durchgeführt. Die Reaktion findet vorzugsweise oberhalb des Siedepunktes des abzuspaltenden ersten Alkohols A statt, um den abgespaltenen ersten Alkohol A direkt aus dem Reaktionsgemisch zu entfernen und somit eine Gleichgewichtsverlagerung zur Produktseite zu begünstigen. Der zweite Alkohol B wird dabei vorzugsweise in einem signifikanten Überschuss, beispielsweise 30 : 1, zum in Schritt a) gebildeten Ester hinzugegeben.

Die basenkatalysierte Umesterung findet vorzugsweise bei einer Temperatur von 20 bis 100 °C statt.

Durch die beschriebe Umesterung wird ein zweites flüssiges Produktgemisch erhalten, welches zumindest den Ester mit dem zweiten Alkohol B, den abgespaltenen ersten Alkohol A und nicht umgesetzte zweite Alkohole B umfasst.

Der in Schritt c1) gebildete zweite Ester wird im nachfolgenden Schritt c2) vom restlichen zweiten flüssigen Produktgemisch abgetrennt. Die Abtrennung wird mittels thermischer Trennung, vorzugsweise Destillation, und/oder mittels Membrantrennung, insbesondere mit den oben beschriebenen Membranmaterialien, durchgeführt. Die entsprechenden Verfahrensbedingungen sind dem Fachmann bekannt.

Es kann vorkommen, dass bei dem in Schritt c2) verwendeten Trennverfahren, insbesondere bei der Destillation, nicht nur der gebildete Ester vom restlichen zweiten flüssigen Produktgemisch abgetrennt wird, sondern auch möglicherweise gebildete Hochsieder, beispielsweise hochsiedende Nebenprodukte, die bei der Umsetzung in Schritt c1) anfallen können. Um diese Hochsieder zu entfernen kann das erfindungsgemäße Verfahren einen Aufreinigungsschritt aufweisen, nämlich die Aufreinigung des in Schritt c1) gebildeten Esters durch Abtrennung des Esters von den vorhandenen Hochsiedern mittels thermischer Trennung, Extraktion, Kristallisation oder Membrantrennung. Bevorzugt wird ein thermisches Trennverfahren, besonders bevorzugt eine weitere Destillation eingesetzt, um die gebildeten Ester aufzureinigen. Die Verfahrensbedingungen sind dem Fachmann bekannt.

Der in Schritt a) hergestellte und in Schritt c) aus dem Permeat abgetrennte und ggf. aufgereinigte Ester wird dann in Schritt d) einer Hydrierung unterworfen. Durch den dabei eingesetzten Wasserstoff wird die Estergruppe gespalten, wodurch der Zielalkohol entsteht und der bei der Esterbildung in Schritt a) angebundenen Alkohol A bzw. den bei der Umesterung angebundenen Alkohol B zurückgewonnen werden kann. Es entsteht demnach bei der Hydrierung ein Alkoholgemisch, welches zumindest den Zielalkohol, den Alkohol A oder B und nicht umgesetzte Ester umfasst.

Ohne Umesterung kann der mit der Hydrierung in Schritt d) zurückgewonnen Alkohol A in einem nachfolgenden Prozessschritt aus dem entstandenen Alkoholgemisch abgetrennt und in die erste Reaktionszone zurückgeführt werden. Wurde eine Umesterung durchgeführt und bei der Hydrierung in Schritt d) der Alkohol B zurückgewonnen, kann dieser nachfolgenden Prozessschritt aus dem entstandenen Alkoholgemisch abgetrennt und in die zweite Reaktionszone zurückgeführt werden.

Die typischen Hydrierbedingungen sind dem Fachmann bekannt und beispielsweise in der EP 1 042 260 A1 offenbart. Die erfindungsgemäße Hydrierung in Schritt d) wird vorzugsweise bei einem Druck von 10 bis 300 bar, weiterhin bevorzugt bei einem Druck von 100 bis 280 bar und besonders bevorzugt bei einem Druck von 150 bis 270 bar durchgeführt. Die Hydriertemperatur liegt vorzugsweise zwischen 100 °C und 300 °C, vorzugsweise zwischen 150 °C und 280 °C und besonders bevorzugt zwischen 180 °C und 220 °C. Bei der Hydrierung kann außerdem ein Abgas entnommen werden um leichtsiedende Komponenten, wie Verunreinigungen wie Stickstoff oder leichtsiedende Nebenprodukte zu entfernen.

Die Hydrierung in Schritt d) findet in Anwesenheit eines heterogenen Katalysatorsystems statt. Typische Katalysatorsysteme sind beispielsweise aus der EP 1 338 557 A1 bekannt. Das heterogene Katalysatorsystem umfasst vorzugsweise ein Metall aus der Gruppe, bestehend aus Kupfer, Rhodium, Ruthenium, Rhenium oder Verbindungen dieser Metalle. Weiterhin sind auch Katalysatorsysteme auf Basis von Kupfer-Chromoxid geeignet. Besonders bevorzugte Katalysatorsystem umfassen Kupfer und/oder Zink als aktive Komponente, die auf ein Trägermaterial aufgebracht sind. Als Trägermaterial eignen sich poröses Siliciumoxid und/oder Aluminiumoxid.

Der zur Hydrierung benötigte Wasserstoff kann direkt als Einsatzstoff bereitgestellt werden. Möglich ist es auch, den Wasserstoff dadurch bereitzustellen, dass ein Wasserstoff-haltiges Gas vorher in einer für den Fachmann bekannten Weise in seine Komponenten aufgetrennt wird und der Wasserstoff zur Hydrierzone geführt wird. Der Wasserstoff kann dabei weiterhin einen gewissen Anteil an Kohlenmonoxid oder anderen Gasen enthalten, weil eine vollständige Auftrennung kaum zu realisieren ist.

Das Alkoholgemisch aus der Hydrierung in Schritt d) wird im nachfolgenden Schritt e) in mindestens einem Trennverfahrensschritt ausgewählt aus der Gruppe, bestehend aus einer thermischen Trennung, beispielsweise Destillation, einer Extraktion, einer Kristallisation oder einer weiteren Membrantrennung, unterworfen, um den in Schritt d) gebildeten Zielalkohol vom restlichen Alkoholgemisch abzutrennen. Das Trennverfahren ist vorzugsweise eine Destillation. Die entsprechenden Verfahrensbedingungen sind dem Fachmann bekannt. Es kann auch eine mehrstufige Destillation durchgeführt werden.

Zudem können in dem mindestens einen Trennverfahrensschritt auch die eingesetzten Alkohole A bzw. B abgetrennt und zur jeweiligen ersten oder zweiten Reaktionszone zurückgeführt werden. Bei der Rückführung kann ein Purgestrom entnommen werde, um beispielsweise Nebenprodukte der Hydrierung inerte Alkane, Aldehyde, Acetale, Ether, Ketone oder Carbons aus dem Verfahren auszuschleusen.

In der Membrantechnik wird zur Charakterisierung der Durchlässigkeit bzw. der Trennleistung einer Membran der Rückhalt R der Membran hinsichtlich einer bestimmten Komponente des Stoffgemisches gemäß nachfolgender Formel (1) definiert:

$$R = 1 - w_{(I)P} / w_{(I)R} \qquad (1),$$

worin $W_{(I)P}$ für den Massenanteil der betrachteten Komponente im Permeat und $W_{(I)R}$ für den Massenanteil der betrachteten Komponente im Retentat der Membran steht. Der Rückhalt kann somit Werte von 0 bis 1 annehmen und wird deswegen gerne in % angegeben. Ein Rückhalt von 0 % bedeutet, dass die betrachtete Komponente ungehindert durch die Membran permeiert, sodass die Massenanteile der Komponenten im Retentat wie im Permeat gleich sind. Andererseits bedeutet ein Rückhalt von 100 %, dass die betrachtete Komponente vollständig von der Membran zurückgehalten wird, was sich technisch jedoch kaum realisieren lässt.

Neben dem Rückhalt ist auch die sogenannte Permeabilität der Membran für die Charakterisierung ihrer Durchlässigkeit gemäß der nachfolgenden Formel (2) maßgeblich:

$$P = m' / (A * TMP) \qquad (2),$$

worin m' für den Massenstrom des Permeats, A für die Fläche der Membran und TMP für den angelegten Transmembrandruck steht. Angegeben wird die Permeabilität in der Regel in der Einheit kg /(h * m$^2$ * bar).Die Permeabilität ist somit eine Kennzahl, die auf die Membranfläche und den eingestellten TMP normiert ist.

Im Hinblick auf die Charakterisierung der Stabilität einer Membran kann eine relative Änderung der Permeabilität $P_{Rel}$ gemäß der nachfolgenden Formel (3) definiert werden:

$$P_{Rel} = P_{t=x} / P_{t=0} \qquad (3),$$

worin $P_{t=x}$ für die Permeabilität zum Zeitpunkt t=x und Pt=o für die ursprüngliche Permeabilität zum Zeitpunkt t=0 steht (möglich ist auch ein anderer zeitlicher Bezugspunkt, mit der Maßgabe, dass t=x > t=y).

Beispiel 1

Umsetzung von Diisobuten (DiB) zu Methyl-3,5,5-trimethylhexanoat (TMH-Ester) mit Membranabtrennung als Katalysator-Recycling.

DiB stellt ein Gemisch bestehend aus den beiden C8-Isomeren 2,4,4-Trimethylpent-1-en und 2,4,4-Trimethylpent-2-en in den Verhältnissen von ungefähr 80:20 dar._Die Versuche wurden in einer durchgehend betriebenen Versuchsanlage durchgeführt, die wie folgt aufgebaut ist.

Im Kern besteht die Anlage aus einem 200 mL Glasautoklav (= Reaktor) der Firma Büchi (bis zu 10 bar bedrückbar). Der Autoklav wird aus einer mit Reaktionslösung gefüllten und mit Argon überschleierten Glasvorlage mittels HPLC Pumpen der Firma Knauer gespeist. Eine weitere HPLC Pumpe der Firma Knauer pumpt aus dem Glasautoklav in den separaten auf bis zu 60 bar bedrückbaren Hochdruckkreislauf, Der Hochdruckkreislauf der Versuchsanlage besteht im Wesentlichen aus einem Flüssigkeitskreislauf, der über eine Kreislaufpumpe betrieben wird, und einer Flachmembrantestzellen sowie der benötigten Sensorik (z.B. Druckmessung, Messung der Temperatur). Der Hochdruckkreislauf wird über eine Kreiselpumpe umgewälzt, um die erforderliche Überströmung der Membranen zu gewährleisten. Aus den Membranmodul wird der durch die Membran dringende Flüssigkeitsstrom als Permeat abgeführt und in einer mit Argon überschleierten Glasvorlagen aufgefangen. Die Permeatmenge wird mittels Waage kontinuierlich erfasst. Die überschüssige Feedmenge (Retentat) wird zurück in den Glasautoklaven geführt. Diese Rückführung erfolgt über einen mechanischen Vordruckregler, mit dem auch der Vordruck der Nanofiltrationsstufe eingestellt wird. Der Kreislauf wird durch einen Thermostaten beheizt, sodass eine definierte Temperatur für die Trennung gewährleistet wird.

Die Versuche wurden bei den folgenden Bedingungen gefahren: 84,5 cm$^2$ aktive Membranfläche, 45 bar Transmembrandruck, 25 °C Separationstemperatur.

Proben können sowohl am Feedstrom zum Hochdruckkreislauf (Feed), aus dem Hochdruckkreislauf (Retentat) und vom Permeat gezogen werden. Zur Analyse der Ausbeute wurden 0,08g der Probe und 0,03g Ethylbenzol (interner Standard) eingewogen und mit 0,25g Acetonitril verdünnt. Die weitere Analyse erfolgte mittels GC-FID. Der Rückhalt an Liganden und Metall wurde Mittels ICP-OES nach vorherigem Aufschluss der Proben von Permeat und Retentat ermittelt. Die Analyse bezüglich des Rückhalts an Säure erfolgte mittels $^{19}$F-NMR.

Die Probenvorbereitung und Analyse alle drei Methoden sind dem Fachmann bekannt.

Als Membranmaterial wurde PEEK eingesetzt. Die erfindungsgemäße PEEK-Aceton Membran wurde analog zur Publikation J. da Silva Burgal et al.; Journal of Membrane Science, Vol. 479 (2015), S. 105-116 (s. auch WO 2015/110843 A1) hergestellt.

Der Inhalt des Glasautoklav wurde gerührt, um eine nahezu ideale Durchmischung zu gewährleisten. Der Druck im Glasautoklav wurde über einen mechanischen Vordruckregler auf 10 bar eingestellt. Der Glasautoklav wird über ein Ölbad mit einer externen im Glasautoklaven angebrachten Temperaturmessung auf der gewünschten Temperatur gehalten.

Zunächst wurde der Glasautoklav und der Hochdruckkreislauf mit einer Grundreaktionsmasse bestehend aus 56 Gew.-% MeOH, 40 Gew.-% DiB, 0,5 Gew.-% 1,2-bis((tert-butyl(pyridin-2-yl)phosphanyl)methyl)benzen (Ligand), 3,4 Gew.-% Aluminiumtriflat (Säure) und 0,1 Gew.-% [Pd(acac)$_{2]}$ (Metall) gefüllt und mit 10bar Argon inertisiert und in Betrieb genommen.

Der Glasautoklav wurde dann auf 100°C Innentemperatur aufgeheizt und der anfängliche Argonstrom auf CO geändert (Reaktionsstart). Über 15 h wurde die Grundreaktionsmasse zum Anfahren des Systems im Kreis gepumpt. Nach 15 h wurde DiB im Feed zu 99% zum TMH-Ester umgesetzt. Nach 15h wurde die Kreis-Fahrweise beendet und das Permeat in separaten Glasbehältern aufgefangen und frische Reaktionslösung (60 Gew.% MeOH und 40 Gew.% DiB) zudosiert. Frischer Katalysator, Ligand oder Säure wurde über den Untersuchungszeitraum von 105h nicht hinzugegeben. Es wurden für die jeweiligen Untersuchungszeiträume die Permeabilität, die Ausbeute des TMH-Esters und die Rückhalte für die Komponenten des Katalysatorsystems mit den oben genannten Methoden bestimmt.

Tabelle 1 Kontinuierliche Umsetzung von DiB zu TMH-Ester

| Zeitraum | T(Glasautoklav) | P | Y(TMH-Ester) * | R(Metall) | R(Ligand) | R(Säurel) |
|---|---|---|---|---|---|---|
| h | °C | kg/m$^2$h$^1$bar$^1$ | % | % | % | % |
| 0-15 | 100 | 0,045 | 99 | ** | | |
| 15-35 | 100 | 0,051 | 84 | 98 | 97 | 97 |
| 35-70 | 90 | 0,049 | 73 | 97 | 98 | 96 |
| 70-90 | 80 | 0,050 | 59 | 98 | 97 | 97 |
| 90-105 | 100 | 0,048 | 84 | 97 | 98 | 96 |
| * Ausbeute zum Ende des jeweils betrachteten Versuchszeitraums ** Keine Rückhalte ermittelt (Anfahren des Systems). | | | | | | |

An Tabelle 1 wird deutlich, dass durch die Membrantrennung mehr als 97% an Metall, Ligand und Säure recycliert wird. Dadurch kann die gleiche Ausbeute für den gewünschten Ester auch ohne weitere Zugabe von frischem Katalysatorsystem auch über längere Zeiträume hinweg erreicht werden. Demnach wird die Aktivität des Katalysatorkomplexes durch diese Methode nicht signifikant beeinflusst.

Beispiel 2

Hydrierung des in Beispiel 1 gewonnen TMH-Esters zu TMH-Alkohol

Das gesammelte Permeat aus Beispiel 1 wurde mittels fraktionierter Destillation aufgetrennt. Zunächst wurde Methanol und nicht umgesetztes Di-isobuten (DiB) entfernt, bevor der Methyl-3,5,5-trimethylhexanoat (TMH-Ester) gewonnen wurde. Der TMH-Ester wurde dann in einem 300 mL Autoklaven mit einem Katalysator, der 15 w% Cu/1,8 w%Cr auf einem Träger (Stuttgarter Masse) umfasste, bei 200°C und 270 bar $H_2$ über 20h zur Reaktion gebracht. Nach Abkühlen und Ablassen des Druckes wurde 1mL Probe gezogen zu der 150 µL Isooctan als interner Standard hinzugefügt, um die Ausbeute und n/iso-Selektivität mittels GC Analyse zu bestimmen. Die Ausbeute betrug 98% an TMH-Alkohol (*n/iso*: 99:1).

Beispiel 3 (Vergleichsbeispiel)

Umsetzung von Diisobuten (DiB) zu Methyl-3,5,5-trimethylhexanoat (TMH-Ester) mit Destillation als Katalysator-Recycling. Die Reaktion wurde wie in Beispiel 1 beschrieben durchgeführt (0,15 L Grundreaktionsmasse bestand aus 56 Gew.-% MeOH, 40 Gew.-% DiB, 0,5 Gew.-% 1,2-bis((tert-butyl(pyridin-2-yl)phosphanyl)methyl)benzen (Ligand), 3,4 Gew.-% Aluminiumtriflat (Säure) und 0,1 Gew.-% [Pd(acac)$_2$] (Metall)).
Nach Reaktionsstart wurden über 4 Stunden stündlich Proben gezogen. Zur Analyse der Ausbeute wurden 0,08g der Probe und 0,03g Ethylbenzol (interner Standard) eingewogen und mit 0,25g Acetonitril verdünnt. Die Analyse erfolgte nach den in Beispiel 1 angegebene Methoden. Nach 4 h wurde die Reaktion beendet und die Reaktionsmasse abgekühlt und der Reaktor entspannt. Durch Anlegen von Vakuum (0,02bar) wurde die Reaktionslösung auf 0,02 L reduziert und vornehmlich Methanol, nicht umgesetztes DiB und TMH-Ester über Kondensation aufgefangen, während das Katalysatorsystem im Glausautoklav verblieb. Eine Analyse einer Probe der im Reaktor verbliebenen Reaktionsmasse mittels GC-FID zeigte nur noch Spuren an Methanol und nicht umgesetzten DiB (<2 Gew.%).
Anschließend wurde der Glasautoklav wieder auf 0,15L zur Durchführung eines zweiten Laufs mit einer Grundreaktionsmasse aus 60 Gew.-% MeOH, 40 Gew.-% DiB befüllt und eine weitere Reaktion durchgeführt.
Nach Reaktionsstart wurden über 4 Stunden stündlich Proben gezogen. Zur Analyse der Ausbeute wurden 0,08g der Probe und 0,03g Ethylbenzol (interner Standard) eingewogen und mit 0,25g Acetonitril verdünnt. Die Analyse erfolgte nach den in Beispiel 1 angegebene Methoden. Nach 4 h wurde die Reaktion beendet und die Reaktionsmasse abgekühlt und der Reaktor entspannt. Tabelle 2 Vergleich der Umsetzung von DiB zu TMH-Ester nach Anwendung von Destillation

| Zeitpunkt der Probenahme | Y(TMH-Ester) erster Lauf | Y(TMH-Ester) zweiter Lauf |
|---|---|---|
| h | % | % |
| 1 | 67 | 11 |
| 2 | 83 | 19 |
| 3 | 88 | 25 |
| 4 | 93 | 27 |

Anhand von Tabelle 2 wird deutlich, dass bei der Destillation zur Abtrennung der Edukte und Produkte vom Katalysator die Reaktivität des Katalysators im zweiten Lauf deutlich verringert ist.

Beispiel 4

Die Herstellung erfolgte analog zu Beispiel 1 mit dem Unterschied, dass Di-n-buten (DnB) eingesetzt wurde.
Das gesammelte Permeat aus Beispiel 1 wurde mittels fraktionierter Destillation aufgetrennt. Zunächst wurde Methanol und nicht umgesetztes Di-n-buten (DnB) entfernt, bevor der erhaltene Ester Methylisononanoate gewonnen wurde. Der erhaltene Ester wurde dann in einem 300 mL Paar Autoklaven mit einem 15 w% Cu/1,8 w%Cr auf Stuttgarter Masse geträgerten Katalysator bei 200°C (auf Innentemperatur geregelt) und 270 bar H2 über 20h bei 500 U/min zur Reaktion gebracht. Nach Abkühlen und Ablassen des Druckes wurde 1mL Probe gezogen zu der 150 µL Isooctan als interner Standard hinzugefügt, um die Ausbeute und n/iso-Selektivität mittels GC Analyse zu bestimmen (Ausbeute: 94% an Isononylalkohol, (*n/iso*: 69:31)).

**Patentansprüche**

1. Verfahren zur Herstellung eines Zielalkohols, wobei das Verfahren die folgenden Schritte umfasst:

   a) Herstellung eines Esters durch Umsetzen (Carbonylieren) eines C2- bis C16-Kohlenwasserstoffs mit mindestens einer Mehrfachbindung mit Kohlenmonoxid und mit einem Alkohol A, welcher ein Mono- oder Polyalkohol (zwei oder mehr OH-Gruppen) mit 1 bis 50 Kohlenstoffatomen oder eine Mischung aus zwei oder mehr Mono- und/oder Polyalkoholen ist und welcher in einem Molverhältnis zum C2- bis C16-Kohlenwasserstoff (Alkohol: C2- bis C16-Kohlenwasserstoff) von 2 bis 20 eingesetzt wird, in Gegenwart eines homogenen Katalysatorsystems, welches zumindest ein Metall der Gruppe 8 bis 10 des Periodensystems der Elemente oder einer Verbindung davon, einen phosphorhaltigen Liganden und eine Säure umfasst, in einer Reaktionszone unter Erhalt eines flüssigen Produktgemisches;
   b) Durchführen einer Membrantrennung zur Abtrennung des homogenen Katalysatorsystems aus dem flüssigen Produktgemisch, wodurch das homogene Katalysatorsystem und zusätzlich nicht umgesetzter Kohlenwasserstoff und/oder nicht umgesetzter Alkohol A im Retentat angereichert werden und der in Schritt a) gebildete Ester im Permeat angereichert wird, wobei als Membranmaterial ein säurestabiles, d. h. ein mindestens 300 h in Anwesenheit der Säure des Katalysatorsystems stabiles Membranmaterial ein OSN-fähiges (Organic Solvent Nanofiltration) Membranmaterial, welches zumindest eine trennaktive Schicht aufweist, eingesetzt wird und wobei das Retentat zur Reaktionszone in Schritt a) zurückgeführt und das Permeat zum nachfolgenden Schritt c) geführt wird;
   c) Abtrennen des in Schritt a) gebildeten Esters aus dem Permeat in mindestens einem Trennverfahrensschritt, ausgewählt aus thermischer Trennung, beispielsweise Destillation, Extraktion, Kristallisation und Membrantrennung;
   d) Hydrieren des in Schritt c) abgetrennten Esters mit Wasserstoff in Gegenwart des heterogenen Katalysatorsystems in einer Hydrierzone unter Erhalt eines Alkoholgemisches, welches zumindest den Zielalkohol, den abgespaltenen Alkohol A und nicht umgesetzte Ester umfasst;
   e) Abtrennen des in Schritt d) gebildeten Zielalkohols in mindestens einem Trennverfahrensschritt, ausgewählt aus thermischer Trennung, beispielsweise Destillation, Extraktion, Kristallisation und Membrantrennung.

2. Verfahren nach Anspruch 1, wobei die trennaktive Schicht aus einem PAEK-Polymer besteht.

3. Verfahren nach Anspruch 2, wobei die trennaktive Schicht aus PEEK besteht, vorzugsweise mit einem Sulfonierungsgrad von weniger als 20%, besonders bevorzugt weniger als 10%.

4. Verfahren nach einem der vorhergehenden Ansprüche, wobei der in Schritt a) eingesetzte Alkohol A ein Mono-oder Polyalkohol (zwei oder mehr OH-Gruppen) mit 1 bis 15 Kohlenstoffatomen, besonders bevorzugt 1 bis 10 Kohlenstoffatomen oder eine Mischung aus zwei oder mehr Mono- und/oder Polyalkoholen ist.

5. Verfahren nach Anspruch 4, wobei der in Schritt a) eingesetzte Alkohol A aus der Gruppe, bestehend aus Methanol, Ethanol, 1-Propanol, 1-Butanol, 1-Pentanol, 1-Hexanol, 2-Propanol, tert-Butanol, 3-Pentanol, 2-Propylheptanol, Cyclohexanol, Phenol und Mischungen daraus, ausgewählt wird.

6. Verfahren nach einem der vorhergehenden Ansprüche, wobei der in Schritt a) eingesetzte C2- bis C16-Kohlenwasserstoff mit mindestens einer olefinischen Doppelbindung ein n- oder iso-Alken mit 2 bis 16 Kohlenstoffatomen ist.

7. Verfahren nach einem der vorhergehenden Ansprüche, wobei das Metall der Gruppe 8 bis 10 des Periodensystems der Elemente oder eine Verbindung davon des homogenen Katalysatorsystems in Schritt a) Palladium oder eine Verbindung davon ist.

8. Verfahren nach einem der vorhergehenden Ansprüche, wobei der phosphorhaltige Ligand des homogenen Katalysatorsystems eine Bidentat-Struktur aufweist.

9. Verfahren nach einem der vorhergehenden Ansprüche, wobei die Umsetzung in Schritt a) bei einem Druck von 5 bis 60 bar durchgeführt wird.

10. Verfahren nach einem der vorhergehenden Ansprüche, wobei die Säure des Katalysatorsystems in Schritt a) eine Brönsted-Säure mit einem pKs ≤ 5, vorzugsweise mit einem pKs ≤ 3 oder eine Lewis-Säure mit einem LAU-Wert von mehr als 25, vorzugsweise mit einem LAU-Wert von 29 ist.

**11.** Verfahren nach einem der vorhergehenden Ansprüche, wobei die Säure des Katalysatorsystems in Schritt a) eine Brönsted-Säure oder eine Lewis-Säure ist, wobei die Brönsted-Säure Perchlorsäure, Schwefelsäure, Phosphorsäure, Methylphosphonsäure oder eine Sulfonsäure ist und wobei die Lewis-Säure Aluminiumtriflat, Aluminiumchlorid, Aluminiumhydrid, Trimethylaluminium, Tris(pentafluorophenyl)boran, Bortrifluorid, Bortrichlorid oder eine Mischungen davon ist.

**12.** Verfahren nach einem der vorhergehenden Ansprüche, wobei Synthesegas vor der Reaktionszone aufgetrennt und das abgetrennte Kohlenmonoxid zur Reaktionszone in Schritt a) und der abgetrennte Wasserstoff zur Hydrierzone in Schritt d) geleitet wird.

**13.** Verfahren nach einem der vorhergehenden Ansprüche, wobei das Verfahren nach der Abtrennung in Schritt c) die folgenden weiteren Schritte aufweist:

c1) Umestern des in Schritt a) gebildeten Esters mit einem zweiten Alkohol B, wobei dieser zweite Alkohol B sich von dem im Schritt a) eingesetzten Alkohol unterscheidet, in einer zweiten Reaktionszone unter Erhalt eines zweiten flüssigen Produktgemischs, welches zumindest den Ester mit dem zweiten Alkohol B, den abgespaltenen ersten Alkohol A und nicht umgesetzte zweite Alkohole B umfasst, wobei der zweite Alkohol B in äquimolarer Menge oder im Überschuss eingesetzt wird;
c2) Abtrennen des in Schritt c1) gebildeten Esters und Auftrennung des restlichen zweiten flüssigen Produktgemisch durch thermische Trennung und/oder mittels Membrantrennung und Rückführung des abgespaltenen ersten Alkohols in die erste Reaktionszone, sowie Rückführung des nicht umgesetzten zweiten Alkohols in die zweite Reaktionszone;
wobei der in Schritt c1) gebildete Ester in der Hydrierung in Schritt d) eingesetzt wird.

**14.** Verfahren nach Anspruch 10 oder 11, wobei der in Schritt e) eingesetzte zweite Alkohol im Vergleich zum ersten Alkohol ein höhersiedender Alkohol ist.

**15.** Verfahren nach einem der Ansprüche 10 bis 12, wobei ein Teil der abgespaltenen ersten Alkohole bereits aus der zweiten Reaktionszone entnommen und zur ersten Reaktionszone zurückgeführt wird.

## Claims

**1.** Process for preparing a target alcohol, wherein the process comprises the following steps:

a) preparing an ester by reacting (carbonylating) a C2 to C16 hydrocarbon having at least one multiple bond with carbon monoxide and with an alcohol A which is a mono- or polyalcohol (two or more OH groups) having 1 to 50 carbon atoms or is a mixture of two or more mono- and/or polyalcohols and which is used in a molar ratio to the C2 to C16 hydrocarbon (alcohol:C2 to C16 hydrocarbon) of 2 to 20, in the presence of a homogeneous catalyst system comprising at least one metal of groups 8 to 10 of the Periodic Table of the Elements or a compound thereof, a phosphorus-containing ligand and an acid, in a reaction zone to obtain a liquid product mixture;
b) performing a membrane separation to separate the homogeneous catalyst system from the liquid product mixture, which enriches the homogeneous catalyst system and additionally unconverted hydrocarbon and/or unconverted alcohol A in the retentate and enriches the ester formed in step a) in the permeate, wherein the membrane material used is an OSN (organic solvent nanofiltration) membrane material which is acid-stable, i.e. stable in the presence of the acid of the catalyst system for at least 300 h, and which has at least one separation-active layer, and wherein the retentate is returned to the reaction zone in step a) and the permeate is routed to the subsequent step c);
c) separating the ester formed in step a) from the permeate in at least one separation process step selected from thermal separation, for example distillation, extraction, crystallization and membrane separation;
d) hydrogenating the ester separated in step c) with hydrogen in the presence of the heterogeneous catalyst system in a hydrogenation zone to obtain an alcohol mixture comprising at least the target alcohol, the eliminated alcohol A and unconverted esters;
e) separating the target alcohol formed in step d) in at least one separation process step selected from thermal separation, for example distillation, extraction, crystallization and membrane separation.

**2.** Process according to Claim 1, wherein the separation-active layer is composed of a PAEK polymer.

3. Process according to Claim 2, wherein the separation-active layer is composed of PEEK, preferably having a degree of sulfonation of less than 20%, particularly preferably less than 10%.

4. Process according to any of the preceding claims, wherein the alcohol A used in step a) is a mono- or polyalcohol (two or more OH groups) having 1 to 15 carbon atoms, particularly preferably 1 to 10 carbon atoms, or a mixture of two or more mono- and/or polyalcohols.

5. Process according to Claim 4, wherein the alcohol A used in step a) is selected from the group consisting of methanol, ethanol, 1-propanol, 1-butanol, 1-pentanol, 1-hexanol, 2-propanol, tert-butanol, 3-pentanol, 2-propylheptanol, cyclohexanol, phenol and mixtures thereof.

6. Process according to any of the preceding claims, wherein the C2 to C16 hydrocarbon having at least one olefinic double bond which is used in step a) is an n- or isoalkene having 2 to 16 carbon atoms.

7. Process according to any of the preceding claims, wherein the metal of groups 8 to 10 of the Periodic Table of the Elements or a compound thereof in the homogeneous catalyst system in step a) is palladium or a compound thereof.

8. Process according to any of the preceding claims, wherein the phosphorus-containing ligand in the homogeneous catalyst system has a bidentate structure.

9. Process according to any of the preceding claims, wherein the reaction in step a) is conducted at a pressure of 5 to 60 bar.

10. Process according to any of the preceding claims, wherein the acid in the catalyst system in step a) is a Brønsted acid having a pKa $\leq$ 5, preferably having a pKa $\leq$ 3, or a Lewis acid having an LAU value of more than 25, preferably having an LAU value of 29.

11. Process according to any of the preceding claims, wherein the acid in the catalyst system in step a) is a Brønsted acid or a Lewis acid, where the Brønsted acid is perchloric acid, sulfuric acid, phosphoric acid, methylphosphonic acid or a sulfonic acid, and where the Lewis acid is aluminium triflate, aluminium chloride, aluminium hydride, trimethylaluminium, tris(pentafluorophenyl)borane, boron trifluoride, boron trichloride or a mixture thereof.

12. Process according to any of the preceding claims, wherein synthesis gas is separated upstream of the reaction zone and the carbon monoxide separated is routed to the reaction zone in step a) and the hydrogen separated to the hydrogenation zone in step d) .

13. Process according to any of the preceding claims, wherein the process after the separation in step c) includes the following further steps:

c1) transesterifying the ester formed in step a) with a second alcohol B, where this second alcohol B differs from the alcohol used in step a), in a second reaction zone to obtain a second liquid product mixture comprising at least the ester with the second alcohol B, the eliminated first alcohol A and unconverted second alcohols B, where the second alcohol B is used in an equimolar amount or in excess;
c2) separating off the ester formed in step c1) and separating the remaining second liquid product mixture by thermal separation and/or by means of membrane separation and recycling the eliminated first alcohol into the first reaction zone, and recycling the unconverted second alcohol into the second reaction zone;
wherein the ester formed in step c1) is used in the hydrogenation in step d).

14. Process according to Claim 10 or 11, wherein the second alcohol used in step e) is a higher-boiling alcohol compared with the first alcohol.

15. Process according to any of Claims 10 to 12, wherein part of the eliminated first alcohol is already withdrawn from the second reaction zone and recycled into the first reaction zone.

**Revendications**

1. Procédé pour la préparation d'un alcool cible, le procédé comprenant les étapes suivantes :

a) préparation d'un ester par transformation (carbonylation) d'un hydrocarbure en C2 à C16 comportant au moins une liaison multiple avec du monoxyde de carbone et avec un alcool A, qui est un monoalcool ou un polyalcool (deux groupes OH ou plus) comportant 1 à 50 atomes de carbone ou un mélange de deux monoalcools et/ou polyalcools ou plus et qui est utilisé en un rapport molaire par rapport à l'hydrocarbure en C2 à C16 (alcool : hydrocarbure en C2 à C16) de 2 à 20, en présence d'un système de catalyseur homogène, qui comprend au moins un métal des groupes 8 à 10 du système périodique des éléments ou un composé correspondant, un ligand contenant du phosphore et un acide, dans une zone de réaction avec obtention d'un mélange liquide de produits ;

b) réalisation d'une séparation par membrane pour la séparation du système de catalyseur homogène du mélange liquide de produits, par laquelle le système de catalyseur homogène et de plus de l'hydrocarbure qui n'a pas réagi et/ou de l'alcool A qui n'a pas réagi sont enrichis dans le rétentat et l'ester formé dans l'étape a) est enrichi dans le perméat, un matériau de membrane stable à l'acide, c'est-à-dire un matériau de membrane stable pendant au moins 300 h en présence de l'acide du système de catalyseur, apte à une NSO (nanofiltration de solvant organique) est utilisé en tant que matériau de membrane, lequel présente au moins une couche séparatrice active, et le rétentat étant recyclé dans la zone de réaction dans l'étape a) et le perméat étant conduit vers l'étape suivante c) ;

c) séparation de l'ester formé dans l'étape a) du perméat dans au moins une étape de procédé de séparation, choisie parmi une séparation thermique, par exemple une distillation, une extraction, une cristallisation et une séparation par membrane ;

d) hydrogénation de l'ester séparé dans l'étape c) avec de l'hydrogène en présence du système de catalyseur hétérogène dans une zone d'hydrogénation avec obtention d'un mélange d'alcools qui comprend au moins l'alcool cible, l'alcool A clivé et de l'ester qui n'a pas réagi ;

e) séparation de l'alcool cible formé dans l'étape d) dans au moins une étape de procédé de séparation, choisie parmi une séparation thermique, par exemple une distillation, une extraction, une cristallisation et une séparation par membrane.

2. Procédé selon la revendication 1, la couche séparatrice active étant constituée d'un polymère de type PAEK.

3. Procédé selon la revendication 2, la couche séparatrice active étant constituée de PEEK, de préférence doté d'un degré de sulfonation inférieur à 20 %, particulièrement préférablement inférieur à 10 %.

4. Procédé selon l'une quelconque des revendications précédentes, l'alcool A utilisé dans l'étape a) étant un monoalcool ou un polyalcool (deux groupes OH ou plus) comportant 1 à 15 atomes de carbone, particulièrement préférablement 1 à 10 atomes de carbone ou un mélange de deux monoalcools et/ou polyalcools ou plus.

5. Procédé selon la revendication 4, l'alcool A utilisé dans l'étape a) étant choisi dans le groupe constitué par le méthanol, l'éthanol, le 1-propanol, le 1-butanol, le 1-pentanol, le 1-hexanol, le 2-propanol, le tert-butanol, le 3-pentanol, le 2-propylheptanol, le cyclohexanol, le phénol et des mélanges correspondants.

6. Procédé selon l'une quelconque des revendications précédentes, l'hydrocarbure en C2 à C16 utilisé dans l'étape a) comportant au moins une double liaison oléfinique étant un n-alcène ou un iso-alcène comportant 2 à 16 atomes de carbone.

7. Procédé selon l'une quelconque des revendications précédentes, le métal des groupes 8 à 10 du système périodique des éléments ou un composé correspondant du système de catalyseur homogène dans l'étape a) étant le palladium ou un composé correspondant.

8. Procédé selon l'une quelconque des revendications précédentes, le ligand contenant du phosphore du système de catalyseur homogène présentant une structure bidentate.

9. Procédé selon l'une quelconque des revendications précédentes, la transformation dans l'étape a) étant réalisée à une pression de 5 à 60 bars.

10. Procédé selon l'une quelconque des revendications précédentes, l'acide du système de catalyseur dans l'étape a) étant un acide de Brönsted doté d'un pKs ≤ 5, de préférence doté d'un pKs ≤ 3 ou un acide de Lewis doté d'une valeur LAU supérieure à 25, de préférence doté d'une valeur LAU de 29.

11. Procédé selon l'une quelconque des revendications précédentes, l'acide du système de catalyseur dans l'étape a) étant un acide de Brönsted ou un acide de Lewis, l'acide de Brönsted étant l'acide perchlorique, l'acide sulfurique, l'acide phosphorique, l'acide méthylphosphonique ou un acide sulfonique et l'acide de Lewis étant le triflate d'aluminium, le chlorure d'aluminium, l'hydrure d'aluminium, le triméthylaluminium, le tri(pentafluorophényl)borane, le trifluorure de bore, le trichlorure de bore ou un mélange correspondant.

12. Procédé selon l'une quelconque des revendications précédentes, du gaz de synthèse étant séparé avant la zone de réaction et le monoxyde de carbone séparé étant conduit vers la zone de réaction dans l'étape a) et l'hydrogène séparé étant conduit vers la zone d'hydrogénation dans l'étape d).

13. Procédé selon l'une quelconque des revendications précédentes, le procédé présentant, après la séparation dans l'étape c), les étapes supplémentaires suivantes :

c1) transestérification de l'ester formé dans l'étape a) avec un deuxième alcool B, ce deuxième alcool B étant différent de l'alcool utilisé dans l'étape a), dans une deuxième zone de réaction avec obtention d'un deuxième mélange liquide de produits, qui comprend au moins l'ester avec le deuxième alcool B, le premier alcool A clivé et des deuxièmes alcools B qui n'ont pas réagi, le deuxième alcool B étant utilisé en quantité équimolaire ou en excès ;
c2) séparation de l'ester formé dans l'étape c1) et séparation du deuxième mélange liquide de produits restant par séparation thermique et/ou au moyen d'une séparation par membrane et recyclage du premier alcool clivé dans la première zone de réaction, ainsi que recyclage du deuxième alcool qui n'a pas réagi dans la deuxième zone de réaction ;
l'ester formé dans l'étape c1) étant utilisé dans l'hydrogénation dans l'étape d).

14. Procédé selon la revendication 10 ou 11, le deuxième alcool utilisé dans l'étape e) étant un alcool à point d'ébullition plus élevé que le premier alcool.

15. Procédé selon l'une quelconque des revendications 10 à 12, une partie des premiers alcools clivés étant déjà prélevée de la deuxième zone de réaction et recyclée dans la première zone de réaction.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2009012323 A1
- EP 3121184 A2
- WO 2015110843 A1
- EP 1042260 A1
- EP 1338557 A1

**In der Beschreibung aufgeführte Nicht-Patentliteratur**

- **JR GAFFEN et al.** *Chem,* vol. 5 (6), 1567-1583
- **J. DA SILVA BURGAL et al.** *Journal of Membrane Science,* 2015, vol. 479, 105-116